(19) Europäisches Patentamt
European Patent Office
Office européen des brevets

(11) Veröffentlichungsnummer: **0 192 056 B1**

(12) # EUROPÄISCHE PATENTSCHRIFT

(45) Veröffentlichungstag der Patentschrift: **26.07.89**

(51) Int. Cl.⁴: **A 61 K 31/35, C 07 D 311/92**

(21) Anmeldenummer: **86100634.4**

(22) Anmeldetag: **18.01.86**

(54) **Verfahren zur Herstellung von Labdan-Derivaten und ihre Verwendung als Arzneimittel.**

(30) Priorität: **26.01.85 DE 3502686**

(43) Veröffentlichungstag der Anmeldung: **27.08.86 Patentblatt 86/35**

(45) Bekanntmachung des Hinweises auf die Patenterteilung: **26.07.89 Patentblatt 89/30**

(84) Benannte Vertragsstaaten: **AT BE CH DE FR GB IT LI NL SE**

(73) Patentinhaber: **HOECHST AKTIENGESELLSCHAFT, Postfach 80 03 20, D-6230 Frankfurt am Main 80 (DE)**

(72) Erfinder: **Dohadwalla, Alihussein N. Dr., Moman Mansion 139 C, Cumbala Hill, Bombay 400 036 (IN)**
Erfinder: **Mandrekar, Sadashiv Shantaram, 8, Darshan Linking Road (Extn.), Santacruz (West) Bombay 400 054 (IN)**
Erfinder: **Dadkar, Nandkumar Keshavrao, Dr., C3 110, Shaivali 358 Minicipal Tenaments, A.G. Khan Road Worli Bombay 400 018 (IN)**
Erfinder: **Khandelwal, Yatendra, Dr., 10C/10 Nitya Nand Nagar 80/83 R.C. Marg, Chembur Bombay 400 071 (IN)**
Erfinder: **Rupp, Richard Helmut, Dr., A84, Meher Apartments Anstey Road, Off Altamound Road Bombay 400 026 (IN)**
Erfinder: **de Souza, Noel John, Dr., 702 Avanti Central Avenue Santacruz (West), Bombay 400 054 (IN)**

(56) Entgegenhaltungen:
**EP-A- 0 116 713**
**EP-A- 0 126 313**
**DE-A- 2 557 784**
**DE-A- 2 640 275**
**DE-A- 2 654 796**
**GB-A- 1 550 410**
**GB-A- 1 589 326**
**US-A- 4 134 986**
**US-A- 4 476 140**

**ARZNEIMITTEL-FORSCHUNG / DRUG RES. Band 31 (II), No. 8, 1981 Aulendorf DE H. METZGER et al.: "The positive inotropic-acting forskolin, a potent adenylatecyclase activator" Seiten 1248-1250**
**JOURNAL OF THE CHEMICAL SOCIETY, PERKIN TRANS. I, 1982, London, GB S.V. BHAT et al.: "Reactions of forskolin, a biologically active diterpenoid from Coleus forskhohlii" Seiten 767-771**
**TETRAHEDRON LETTERS, No. 19, 1977, Pergamon Press, Oxford, GB S.V. BHAT et al.: "Structures and**

(56) Entgegenhaltungen: (Fortsetzung)
**stereochemistry of new labdane diterpenoids from coleus forskohlii brig" Seiten 1669-1672**
**JOURNAL OF THE MEDICINAL CHEMISTRY, Band 26, 1983 (Washington, US) K. SEAMAN et al.: "Structure-activity relationships for activation of adenylate cyclase by the diterpene forskolin and derivatives" Seiten 436-439**
**JOURNAL OF MEDICINAL CHEMISTRY, Band 26, 1983; (Washington, US) S. BHAT et al.: "The Antihypertensive and Positive Inotropic Diterpene forskolin: Effects of Structural Modifications on its Activity" Seiten 486-492**
**CHEMICAL ABSTRACTS, Band 89, Nr. 3, 1978, Columbus, Ohio, USA J.S. TANDON et al.: "Structure of coleonol, a biologically active diterpene from coleus forskohlii" Seite 671, Zusammenfassung-Nr. 24556z**
**CHEMICAL ABSTRACTS, Band 89, 1978, Columbus, Ohio, USA J.S. TANDON et al.: "Structures of three new diterpenes, coleonol B, coleonol C and deoxycoleonol isolated from coleus forskohlii" Seiten 594, 595, Zusammenfassung-Nr. 163779n**

**EP 0 192 056 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft oxygenierte Labdan-Derivate, ein Verfahren zu deren Herstellung und ihre Verwendung als Antiphlogistika und Analgetika.

Das Labdanderivat Forskolin wird im Indischen Patent Nr. 143 875 und der entsprechenden DE-OS 2 557 784 beschrieben und besitzt die folgende Strukturformel I:

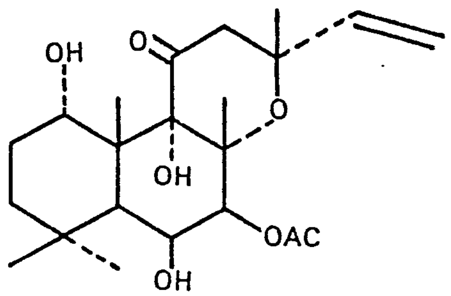

I

(7β-Acetoxy-8,13-epoxy-1α-6β,9α-trihydroxy-labd-14-en-11-on)

1,9-Didesoxyforskolin ist in Tetrahedron-Letters Nr. 19, S. 1669–1672 (1977) beschrieben worden und besitzt die folgende Strukturformel II

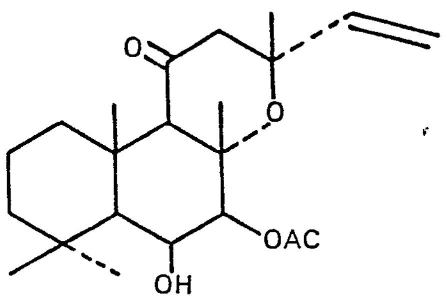

II

(7β-Acetoxy-1,9-didesoxy-8,13-epoxy-6β-hydroxy-labd-14-en-11-on)

Weitere, natürlich vorkommende Forskoline werden in der vorgenannten Veröffentlichung und im J. Med. Chem., 26, 486 (1983) beschrieben; die Verbindung Coleforsin wird im Indischen Patent Nr. 147 030 entsprechend DE-OS 2 640 275 beschrieben; synthetisch modifizierte Forskoline werden in J. Chem. Soc., Perkin Trans., 1 767 (1982), J. Med. Chem., 26 486 (1983) und im Indischen Patent Nr. 147 007 und der entsprechenden DE-OS 2 654 796 beschrieben.

Oxygenierte Labdan-Derivate, für die Forskolin als Beispiel steht, weisen interessante pharmakologische Eigenschaften auf, so dass sie als Arzneimittel bei der Behandlung von Herz- und Kreislauferkrankungen, Hypertonie, Glaukom, Allergie, Bronchokonstriktion und als Immunstimulantien (siehe z.B. DE-OS 3 314 999) in Frage kommen.

Überraschenderweise wurde nun gefunden, dass oxygenierte Labdan-Derivate entzündungshemmende und analgetische Eigenschaften aufweisen.

Es existiert ständig ein Bedürfnis an sicheren und wirksamen Antiphlogistika. Bei der Entzündung handelt es sich um einen Krankheitsprozess, für den Rötung, Fieber, Schwellung und Schmerzen charakteristisch sind. Die am häufigsten vorkommende und schwerste der entzündlichen Erkrankungen ist die Arthritis in ihren unterschiedlichen Formen. Zur Entzündung kann es ferner bei traumatischen Verletzungen und bei Infektionen kommen, weshalb bei deren Behandlung häufig Antiphlogistika eingesetzt werden. Die Anwendbarkeit der meisten im Handel befindlichen Antiphlogistika ist wegen ihrer Toxizität und ungünstigen Nebenwirkungen begrenzt. Viele rufen eine Magenreizung und andere Wirkungen wie eine Veränderung der Blutzellen und des Zentralnervensystems hervor. Adrenocorticosteroide führen zur Magenreizung und Suppression der normalen Nebennierenfunktion.

Die vorliegende Erfindung ist das Ergebnis der Bemühungen um die Entwicklung neuer antiarthritischer Substanzen mit guter antiphlogistischer Wirkung und minimalen Nebenwirkungen.

Neben den antiphlogistischen Eigenschaften haben einige Verbindungen gemäss dieser Erfindung eine analgetische Wirkung gezeigt. Diese Eigenschaft ist bei der Behandlung der Arthritis oder verwandter Erkrankungen erwünscht; derartige Verbindungen können allerdings ausschliesslich zur Schmerzbekämpfung eingesetzt werden.

Die vorliegende Erfindung betrifft daher die Verwendung von oxygenierten Labdan-Derivaten, wie zum Beispiel Forskolin und 1,9-Didesoxyforskolin, als Antiphlogistika und Analgetika.

Die vorliegende Erfindung betrifft die Verwendung von oxygenierten Labdan-Derivaten der Formel III

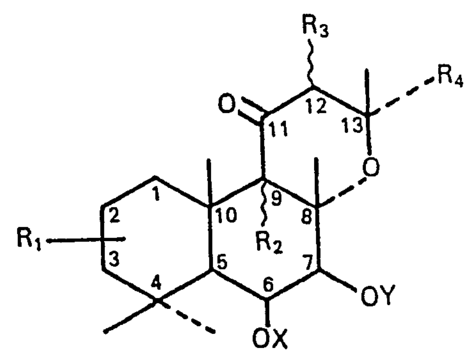

III

worin $R_1$, $R_2$ und $R_3$ gleich oder verschieden sind und H, OH oder den Rest –OCOR bedeuten, wobei R für $C_1$–$C_3$-Alkyl steht und $R_1$ eine oder mehrere der Positionen 1–3 der Formel einnehmen kann, $R_4$ –CHO, $-CH=CH_2$, $-\underset{\diagdown O \diagup}{CH-CH_2}$, –CHOH-$CH_2OH$ bedeutet und X und Y H oder $-\overset{O}{\overset{\|}{C}}R$ bedeuten, wobei R die vorgenannten Bedeutungen aufweist, zur Herstellung von Arzneimitteln zur Behandlung von Entzündungskrankheiten und Schmerzzuständen bei Säugetieren einschliesslich Menschen. Die bevorzugten Substanzen der

Erfindung sind Forskolin der Formel I und 1,9-Didesoxyforskolin der Formel II.

Das Verfahren, nach dem 1,9-Didesoxyforskolin aus der Pflanze Coleus forskohlii isoliert wird, ist bisher nicht ausführlich beschrieben worden. Seine Isolierung wurde in Tetrahedron Letters 19, S. 1669–1672 (1977) kurz erwähnt.

Folgendes Verfahren eignet sich zur Herstellung von 1,9-Didesoxyforskolin. Man verwendet vorzugsweise die getrockneten und gemahlenen Wurzeln von Coleus Forskohlii und isoliert die Verbindung gemäss dem folgenden Schema:

Schema

Getrocknete, gemahlene Pflanzenteile

Lösungsmittel-Extraktion

Extraktlösung

Konzentration

Rückstand

1) Behandlung mit organischen Lösungsmitteln
2) Konzentration der Lösungen

Rückstand

1) Säulenchromatographie
2) Kristallisation

1,9-Didesoxyforskolin

Zum Extrahieren der gewünschten Substanz aus Coleus forskohlii verwendet man vorzugsweise aromatische Kohlenwasserstoffe oder halogenierte aliphatische Kohlenwasserstoffe mit 1 bis 3 Kohlenstoffatomen und bis zu 3 Halogenatomen, vorzugsweise bis zu 3 Chloratomen, oder einen niederen Alkanol mit 1 bis 6 Kohlenstoffatomen. Unter diesen Extraktionsmitteln sind Benzol, Toluol, Xylol, Methylenchlorid, Chloroform, Methanol und Äthanol bevorzugt. Die Extraktionsmittel werden vorzugsweise im Gewichtsverhältnis von 2:1 bis 10:1 bezogen auf die zu isolierende Substanz verwendet. Die Extraktion kann bei Temperaturen erfolgen, die zwischen Zimmertemperatur und dem Siedepunkt des zur Extraktion verwendeten Lösungsmittels, vorzugsweise bei 30°–40 °C liegen. Die Extraktlösung wird unter vermindertem Druck, vorzugsweise im Vakuum eingedampft. Der Rückstand wird bis zur vollständigen Extraktion weiter mit einem organischen Lösungsmittel wie beispielsweise Petroleumäther behandelt.

Der Petroleumäther-Extrakt wird dann, vorzugsweise im Vakuum, bis auf einen Rückstand konzentriert, der mittels Säulenchromatographie über Kieselgel und durch Kristallisation gereinigt wird, zum gewünschten 1,9-Didesoxyforskolin.

Zum Nachweis der antiphlogistischen Wirkung der Verbindungen der Formel III, für die eine gute Korrelation zur Wirksamkeit beim Menschen besteht, wurde der akute Carrageenin-induzierte Rattenpfotenödem-Test, der Krotonöl-induzierte lokale Entzündungs-Test und der durch Adjuvantien induzierte Arthritis-Test bei Ratten verwendet.

a) Akuter Carrageenin-induzierter Rattenpfotenödem-Test

Männliche Charles-Foster-Ratten (120–150 g) erhalten 18 Stunden lang kein Futter, sondern nur Wasser ad libitum. Die in 0,5% Carboxymethylzellulose (CMC) suspendierte Testsubstanz wird intraperitoneal verabreicht. Die Kontrollgruppe erhält 0,5% CMC-Suspension. 0,05 ml der 0,5%igen Carrageenin-Suspension werden subkutan in die Plantarregion der linken Hinterpfote injiziert. Das Pfotenvolumen wird vor der Carrageenin-Injektion und 3 und 6 Stunden nach der Injektion mit einem Maclab-Volumendifferenzmesser bestimmt. Die prozentuale Abnahme des Pfotenvolumens wird nach der folgenden Formel berechnet:

$$\frac{\text{Mittleres Ödemvolumen (Kontrollgruppe)} - \text{Mittleres Ödemvolumen (Testgruppe)}}{\text{Mittleres Ödemvolumen (Kontrollgruppe)}} \times 100$$

= Abnahme des Pfotenvolumens (in %)

Der $ED_{50}$-Wert wird aus der Dosiswirkungskurve berechnet. Jede Gruppe besteht aus sechs Tieren.

b) Krotonöl-induzierter lokaler Entzündungstest bei Ratten

Unter Äthernarkose werden Ratten mit einem Gewicht zwischen 145 und 160 g (Charles-Foster-Stamm) mit Hilfe einer Mikroglasspritze und einer stumpfen 20G-Kanüle in die Vorder- und Rückseite des Ohres 0,05 ml Krotonöl-Irritans (2,0 ml Pyridin + 0,5 ml Aqua dest. + 7,4 ml Diäthyläther + 0,1 ml Krotonöl) verabreicht (Tonelli, G., Thibault, L. und Ringer, I., Endocrinology 77, 625–634, (1965)).

Das rechte unbehandelte Ohr dient als Kontrolle. Die mit dem Medikament behandelte Gruppe erhält verschiedene Konzentrationen der Substanz zusammen mit dem Irritans. Die Tiere werden vier Stunden später getötet. Die Ohren werden entfernt und mit einem Korkenbohrer mit 8 mm Durchmesser gestanzt. Die ausgestanzten

Teile werden sofort gewogen. Die Gewichtszunahme des Ohres wird durch die Differenz des Gewichts zwischen linkem und rechtem Ohr ermittelt.

Die äusserliche antiphlogistische Wirkung der Testsubstanz wird im Prozent Hemmung der Gewichtszunahme des mit dem Irritans behandelten Ohres nach folgender Formel ausgedrückt:

$$\frac{\text{Gewichtszunahme des mit Irritans behandelten Ohres} - \text{Gewichtszunahme des mit Irritans + Medikament behandelten Ohres}}{\text{Gewichtszunahme des mit Irritans behandelten Ohres}} \times 100$$

= Hemmung (in %)

Der $ED_{50}$-Wert wird aus der Dosiswirkungskurve berechnet. Jede Gruppe besteht aus sechs Tieren.

c) Beginnende und bestehende, durch Adjuvantien induzierte Arthritis bei Ratten

Weiblichen Charles-Foster-Ratten (180–200 g) werden in die Schwanzbasis 0,1 ml einer 1%-Suspension von Mycobacterium tuberculum in Mineralöl injiziert. Den nicht-arthritischen Kontrollen wird Mineralöl injiziert. Bei jeder Ratte wird vom 7. Tag täglich bis zum Tag 21 mittels Maclab-Volumendifferenzmesser das Volumen der rechten und linken Hinterpfote gemessen. Im Falle einer beginnenden Arthritis wird 21 Tage lang einmal täglich die Testsubstanz, suspendiert in 0,5% Carboxymethylzellulose (CMC), intraperitoneal injiziert. Bei nachgewiesener Arthritis wird stattdessen die in 0,5% CMC suspendierte Testsubstanz nach voller Ausbildung der Arthritis, d.h. 15 Tage nach der Injektion des Adjuvans, also vom Tag 15 bis 21, einmal täglich intraperitoneal injiziert.

Die prozentuale Hemmung des Pfotenvolumens bei der beginnenden Arthritis wird wie folgt bestimmt:

$$\frac{\text{Mittlere Pfotenvolumenänderung, unbehandelt} - \text{Mittlere Pfotenvolumenänderung nach Behandlung}}{\text{Mittlere Pfotenvolumenänderung, unbehandelt}} \times 100$$

= Hemmung des Pfotenvolumens (in %)

Die prozentuale Hemmung des Pfotenvolumens bei der bestehenden Arthritis wird wie folgt ermittelt:

$$\frac{\text{Mittleres Pfotenvolumen, unbehandelt} \quad \text{Mittleres Pfotenvolumen, nach Behandlung}}{\text{Mittleres Pfotenvolumen, unbehandelt}} \times 100$$

= Hemmung des Pfotenvolumens (in %)

Analgetische Wirkung

Essigsäure-Krampftest

Männlichen Mäusen (20–25 g) wird eine in 0,5% Carboxymethylzellulose suspendierte Testsubstanz subkutan injiziert. Die Kontrollgruppe erhält ausschliesslich 0,5% CMC. Nach 30 Minuten werden intraperitoneal 0,2 ml 3% Essigsäure injiziert. Die Tiere werden 15 Minuten lang auf ein charakteristisches Streck- oder Krampf-Syndrom beobachtet, das den durch Essigsäure ausgelösten Schmerz anzeigt. Jede Gruppe besteht aus sechs Tieren.

In der Testgruppe werden Mäuse mit bis zu 50% des mittleren Kontroll-Krampfwertes als geschützt betrachtet

(Blumberg et al., Proc. Soc. Exp. Biol. Med. 118, 763–766 (1965)). Die prozentuale analgetische Wirkung wird wie folgt berechnet:

$$\text{analgetische Wirkung (in \%)} = \frac{\text{Anzahl geschützter Mäuse}}{\text{Gesamtzahl der Mäuse}} \times 100$$

Der $ED_{50}$-Wert wird aus der Dosiswirkungskurve errechnet.

Die bei diesen Tests mit den Substanzen der Erfindung erzielten Resultate sind am Beispiel von Forskolin und 1,9-Didesoxyforskolin in der nachfolgenden Tabelle dargestellt.

Antiphlogistische und analgetische Wirkung von Forskolin und 1,9-Didesoxyforskolin

| Wirkung | Forskolin | 1,9-Didesoxy-forskolin |
|---|---|---|
| a) Äusserlich Krotonöl-induzierte Rattenohr-Entzündung | $ED_{50}$ : 1,15 mg/Ohr | $ED_{50}$ : 1,6 mg/Ohr |
| b) Systemisch Carrageenin-induziertes Rattenpfotenödem | $ED_{50}$ : 4,80 mg/kg i.p. | $ED_{50}$ : 2,2 mg/kg i.p. |
| (c) Adjuvans-induzierte Arthritis bei Ratten | | |
| Beginnende Arthritis | 2,5 mg/kg i.p.: 23% Hemmung | 3,0 mg/kg i.p.: 62% Hemmung |
| Bestehende Arthritis | 2,4 mg/kg i.p.: 27% Hemmung | 3,0 mg/kg i.p.: 30% Hemmung |
| Analgetische Wirkung Essigsäure-induzierter Krampf bei Mäusen | | $ED_{50}$ : 0,9 mg/kg subkutan |

Die erfindungsgemäss verwendeten Verbindungen können also zur Behandlung der Arthritis oder anderer entzündlicher Erkrankungen oder zur Schmerzlinderung bei Säugetieren verwendet werden. Sie können auf jede konventionelle Weise verabreicht werden, entweder als alleinige therapeutische Substanzen oder in Form einer Kombination von therapeutischen Substanzen. Sie können alleine verabreicht werden, doch es ist üblich, sie je nach Darreichungsform und üblicher pharmazeutischer Praxis mit Träger- und/oder Hilfsstoffen zu verabreichen.

Übliche Darreichungsformen sind die orale Verabreichung in fester Form wie Kapseln, Tabletten und Pulvern oder in flüssiger Form wie Elixiere, Sirupe und Suspensionen; die Verabreichung kann auch parenteral in steriler flüssiger Form erfolgen.

Gelatine-Kapseln enthalten den wirksamen Bestandteil und pulverisierte Träger wie Lactose, Saccharose, Mannitol, Stärke, Zellulosederivate, Magnesiumstearat, Stearinsäure oder ähnliches. Mit den gleichen Streckmitteln können auch Tabletten gepresst werden. Sowohl Tabletten wie Kapseln können als Retardform hergestellt werden, bei der das Medikament über mehrere Stunden freigesetzt wird. Die gepressten Tabletten können mit Zucker oder einem Überzug beschichtet werden, um den unangenehmen Geschmack zu überdecken und das Medikament vor der Atmosphäre zu schützen, sie können aber auch als magensaft-resistente Tabletten hergestellt werden, die selektiv im Magen-Darmtrakt aufgelöst werden.

Die flüssigen Darreichungsformen zur oralen Anwendung können Farb- und Aromastoffe enthalten, um für den Patienten angenehmer zu sein.

Generell sind Wasser, ein geeignetes Öl, Kochsalzlösung, wässerige Dextrose (Glukose) und verwandte Zuckerlösungen und Glykole wie Propylenglykol oder Polyäthylenglykole geeignete Träger für parenterale Lösungen. Die Lösungen zur parenteralen Verabreichung enthalten vorzugsweise ein wasserlösliches Salz des Wirkstoffes, geeignete Stabilisatoren sind erforderlichenfalls Puffersubstanzen. Geeignete Stabilisatoren sind Antioxidantien wie Natriumbisulfit, Natriumsulfit oder Ascorbinsäure alleine oder als Kombination. Verwendet werden können auch Zitronensäure und deren Salze sowie Natrium-EDTA. Ausserdem können parenterale Lösungen Konservierungsstoffe wie Benzalkoniumchlorid, Methyl- oder Propylparaben oder Chlorbutanol enthalten.

Geeignete pharmazeutische Trägerstoffe werden in Remington's Pharmaceutical Sciences, E. W. Martin, als Standard-Quellentext auf diesem Gebiet beschrieben.

Die erfindungsgemäss verwendeten Verbindungen können oral in einer Dosiseinheit von 0,5–50 mg/kg und parenteral in einer Dosiseinheit von 0,05–10 mg/kg Körpergewicht verabreicht werden.

Die folgenden Beispiele illustrieren diese Erfindung.

Beispiel 1

Die getrockneten und pulverisierten Wurzeln von Coleus forskohlii (4 kg Trockengewicht) wurden dreimal nacheinander (20 l, 16 l, 16 l) bei einer Temperatur von 35–40 °C mit einer Gesamtmenge von 52 l Benzol extrahiert. Der Abschluss der Extraktion wurde dadurch geprüft, dass auf einer TLC-Platte nach den aufeinanderfolgenden Extraktionen ein negativer Farbtest für die Anwesenheit von Terpenoiden (Vanillin – Schwefelsäure oder Anisaldehyd – Schwefelsäure-Spray) beobachtet wurde.

Die Benzol-Extrakte wurden im Vakuum bei einer Temperatur unter 40 °C bis zur Trockenheit eingedampft. Der Rückstand (0,2 kg) wurde dreimal (mit jeweils 2 l) mit einer Gesamtmenge von 6 l Petroleumäther verrieben (Siedepunkt 60–80 °C). Die Petroleumäther-Schicht wurde abgetrennt und bei einer Temperatur unter 40 °C im Vakuum bis zur Trockenheit eingedampft. Mittels Säulenchromatographie über Kieselgel wurde aus dem Rückstand (52 g) rohes 1,9-Didesoxyforskolin gewonnen, das aus Äthylacetat-Petroleumäther (1:9) kristallisiert wurde und 1,9-Didesoxyforskolin lieferte (1,2 g) mit einem Schmelzpunkt von 162–65 °C.

Beispiel 2

Getrocknete und gemahlene Wurzeln (12 kg) von Coleus forskohlii wurden bis zur ausgiebigen Extraktion mit jeweils 25 l Chloroform extrahiert. Es wurden 75 l Chloroform verwendet. Die kombinierten Chloroform-Extrakte wurden filtriert und im Vakuum evaporiert. Der Rückstand (ca. 300 g) wurde dreimal mit jeweils 1,5 l Petroleumäther verrührt und filtriert. Das bis zur Trockenheit eingedampfte Filtrat ergab einen gummiartigen Rückstand (ca. 125 g). Der Rückstand wurde weiter, wie in Beispiel 1 beschrieben, behandelt und ergab 3,58 g 1,9-Didesoxyforskolin mit einem Schmelzpunkt von 162–65 °C.

**Patentansprüche**

1. Verwendung von Verbindungen der Formel III

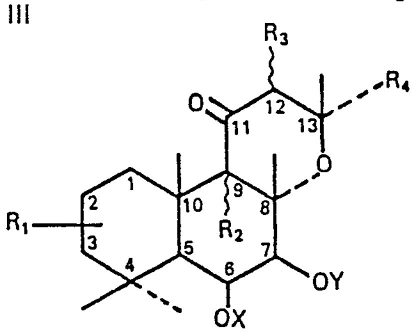

III

worin $R_1$, $R_2$ und $R_3$ unabhängig voneinander H, OH oder $-O\overset{O}{\overset{\|}{C}}R$ bedeuten, wobei R $C_1$–$C_3$-Alkyl darstellt und der Substituent $R_1$ in einer oder mehreren Positionen 1–3 der Formel stehen kann, $R_4$ die Reste $-CHO$, $-CH=CH_2$, $-\underset{\diagdown O \diagup}{CH_2-CH_2}$ oder $-CHOHCH_2OH$ bedeuten und X und Y für H oder den Rest $-\overset{O}{\overset{\|}{C}}R$ stehen, wobei R die oben genannte Bedeutung hat, zur Herstellung von Arzneimitteln, zur Behandlung von Entzündungskrankheiten und Schmerzzuständen bei Säugetieren einschliesslich Menschen.

2. Verwendung einer Verbindung der Formel III gemäss Anspruch 1, worin $R_1$ die 1-OH-Gruppe darstellt, $R_2$ OH, $R_3$ und X Wasserstoff, Y den Rest $-\overset{O}{\overset{\|}{C}}CH_3$ und $R_4$ die Vinylgruppe darstellt.

3. Verwendung einer Verbindung der Formel III nach Anspruch 1, worin $R_1$, $R_2$, $R_3$ und X Wasserstoff, Y den Rest $-\overset{O}{\overset{\|}{C}}CH_3$ und $R_4$ die Vinylgruppe bedeuten.

**Revendications**

1. Utilisation de composés de formule III

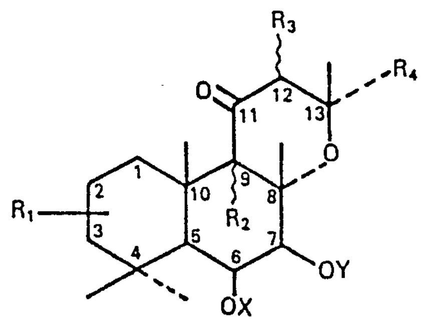

III

dans laquelle $R_1$, $R_2$ et $R_3$ représentent, indépendamment les uns des autres, H, OH ou $-\overset{O}{\overset{\|}{C}}OR$, R représentant un groupe alkyle en $C_1$–$C_3$, et le substituant $R_1$ pouvant occuper une ou plusieurs positions 1–3 de la formule; $R_4$ représente les radicaux $-CHO$, $-CH=CH_2$, $-\underset{\diagdown O \diagup}{CH_2-CH_2}$ ou $-CHOHCH_2OH$; et X et Y représentent H ou le radical $-\overset{O}{\overset{\|}{C}}R$, R ayant la signification donnée ci-dessus; pour la fabrication de médicaments pour le traitement de maladies inflammatoires et d'états douloureux, chez des mammifères, l'homme y compris.

2. Utilisation d'un composé de formule III selon la revendication 1, dans lequel $R_1$ représente le groupe 1–OH, $R_2$ représente OH, $R_3$ et X représentent un atome d'hydrogène; Y représente le radical $-\overset{O}{\overset{\|}{C}}CH_3$; et $R_4$ représente le groupe vinyle.

3. Utilisation d'un composé de formule III selon la revendication 1, dans lequel $R_1$, $R_2$, $R_3$ et X représentent un atome d'hydrogène, Y représente le radical $-\overset{O}{\overset{||}{C}}CH_3$, et $R_4$ représente le groupe vinyle.

**Claims**

1. The use of a compound of the formula III

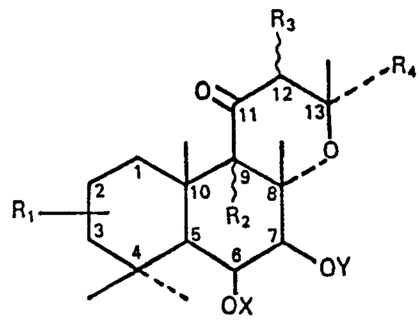

III

wherein $R_1$, $R_2$ and $R_3$ independently of one another denote H, OH or $-O\overset{O}{\overset{||}{C}}R$, where R represents $C_1$–$C_3$-alkyl and the substituent $R_1$ may be in one or more positions 1–3 of the formula, $R_4$ denotes the radicals $-CHO$, $-CH=CH_2$, $-\underset{O}{\underbrace{CH_2-CH_2}}$ or $-CHOHCH_2OH$ and X and Y stand for H or the radical $-\overset{O}{\overset{||}{C}}R$, where R has the abovementioned meaning, for the preparation of pharmaceuticals for the treatment of inflammatory diseases and pain states in mammals including humans.

2. The use of a compound of the formula III as claimed in claim 1, wherein $R_1$ represents the 1–OH group, $R_2$ represents OH, $R_3$ and X represent hydrogen, Y represents the radical $-\overset{O}{\overset{||}{C}}CH_3$ and $R_4$ represents the vinyl group.

3. The use of a compound of the formula III as claimed in claim 1, wherein $R_1$, $R_2$, $R_3$ and X denote hydrogen, Y denotes the radical $-\overset{O}{\overset{||}{C}}CH_3$ and $R_4$ denotes the vinyl group.